# EUROPEAN PATENT SPECIFICATION

(11) **EP 0 787 178 B1**
(45) Date of publication and mention of the grant of the patent: **09.01.2002**
(21) Application number: 95937666.6
(22) Date of filing: 27.10.1995
(51) Int. Cl.: C12M 3/06, C12M 1/12

(54) **MULTI-LAYER GAS-PERMEABLE CONTAINER FOR THE CULTURE OF ADHERENT AND NON-ADHERENT CELLS**
MEHRSCHICHTIGER GASDURCHLÄSSIGER BEHÄLTER ZUR ZUCHTUNG VON ADHÄRENTEN UND NICHT-ADHÄRENTEN ZELLEN
RECEPTACLE MULTICOUCHE PERMEABLE AU GAZ DESTINE A LA CULTURE DE CELLULES ADHERENTES ET NON ADHERENTES

(30) Priority: 28.10.1994 US 330717
(43) Date of publication of application: 06.08.1997
(73) Proprietor: BAXTER INTERNATIONAL INC., Deerfield, IL 60015-4633 (US)
(72) Inventor: SMITH, Sidney, T., Lake Forest, IL 60045 (US); BACEHOWSKI, David, V., Wildwood, IL 60030 (US); KOLANKO, William, Grayslake, IL 60030 (US); ROSENBAUM, Larry, Gurnee, IL 60031 (US); SMITH, Stephen, L., Arlington Heights, IL 60005 (US); LING, Michael, T.K, Vernon Hills, IL 60061 (US); WOO, Lecon, Libertyville, IL 60048 (US); BENDER, James, G, Lindenhurst, IL 60046 (US)
(74) Representative: MacGregor, Gordon
(86) International application number: PCT/US95/13943
(87) International publication number: WO 96/13573

(56) References cited:
- WO-A-90/03427
- US-A- 4 717 668
- US-A- 4 879 232
- US-A- 4 939 151
- US-A- 4 968 624
- DATABASE WPI Section Ch, Week 9118 Derwent Publications Ltd., London, GB; Class A92, AN 91-127852 & JP,A,03 065 177 (NISSHO KK) , 20 March 1991

## Description

### Technical Field

This invention relates to multi-layer films, and containers formed therefrom for the in vitro culture of cells. Specifically, the invention is directed to a multi-layer, flexible, gas permeable container having an inner growing surface of polystyrene, which is conducive to the culture of cells.

### Background Art

There are two major types of cells grown in vitro: suspension cells (anchorage-independent cells); and adherent cells (anchorage-dependent cells). Suspension or anchorage-independent cells can multiply, in vitro, without being attached to a surface. In contrast, adherent cells require attachment to a surface in order to grow in vitro. Additionally, some non-adherent cells grow best on a surface that promotes adherent cell growth.

It is known to grow adherent cells, in vitro, in polystyrene flasks. Polystyrene is the most common type of plastic used in the manufacture of rigid, gas impermeable cell culture flasks or plates. It is thought that polystyrene promotes the growth of adherent cells because of its ability to maintain electrostatic charges on its surface which attract oppositely charged proteins on the cell surfaces. However, to date, the available polystyrene culture container have been of the rigid flask or plate type because polystyrene is known in the art as a rigid, gas-impermeable plastic.

Cells are commonly cultured in a growth medium within polystyrene or other containers placed in enclosed incubators. In addition to providing a certain degree of isolation from pathogens, the incubators maintain a constant temperature, usually 37°C, and a constant gas mixture. The gas mixture must be optimized for a given cell type, and be controlled for at least two parameters: (1) partial pressure of oxygen (pO₂) to serve the aerobic needs of the cells, and (2) partial pressure of carbon dioxide (pCO₂) to maintain the pH of the growth medium. Since the known types of rigid cell culture containers are gas impermeable, their lids or caps are not sealed onto the containers. Rather, they are offset sufficiently to allow gas exchange through a gap or vent between the cap and the container. Such a container is disadvantageous for clinical uses because the vent might allow contamination of the culture or lead to accidents involving biohazardous agents.

In addition to polystyrene flasks, others have constructed flexible, breathable containers for containing adherent cells to be grown in vitro. For example, the commonly assigned U.S. Patent No. 4,939,151 provides a gas-permeable bag with at least one access port. This allows for a closed system (ie., one without a vent). The bag disclosed in the '151 Patent is constructed from two side walls. The first side wall is made of ethylene-vinyl acetate ("EVA") which may be positively or negatively charged. The second side wall is constructed from a gas permeable film such as ethylene-vinyl acetate or a polyolefin. The first side wall is sealed to the second side wall along their edges. While EVA can hold an electrostatic charge, the charge has the undesirable tendency to decay over time. Eventually, the decay of the charge on EVA will render the container ineffective for growing adherent cells. Rigid styrene flasks with an electrostatic charge are known, and show less of a tendency to lose charge over time.

It has been found that the cell growth rate within a sealed container may be influenced by the gas permeability characteristics of the container walls. The optimal gas requirements, however, vary by cell type and over the culture period. Thus, it is desirable to be able to adjust the gas permeability of the container. The polystyrene flask, and the flexible flask which is entirely constructed from a monofilm, do not provide for such adjustability.

### Summary of the Invention and Objects

The present invention provides a multi-layer, coextruded film according to claim 1 suitable for producing gas-permeable cell culture bags. The film has an ultra-thin first layer of polystyrene having a thickness from about 2.54µm (0.0001 inches) to about 25.4µm (0.0010 inches). The film has a second layer adhered to the first layer preferably made of a polyolefin. The polyolefin acts as a flexible substrate for the polystyrene to provide a flexible, gas permeable film. Thus, the second layer is sometimes referred to as the substrate layer.

The film may also have an adhesive tie layer interposed between the first and second layers. The film may also have one or more additional outer layers of polyolefin (such as polypropylene or polyethylene) to provide strength and scratch resistance, as well as additional tie layers interposed between these additional layers.

The film most preferably has the following physical characteristics: (1) a mechanical modulus of between about 6.9 x 10⁷ and 2.1 x 10⁸Pa (10,000 and 30,000 psi) (ASTM D 790); (2) an oxygen permeability within the range of about 9-15 Barrers; (3) a carbon dioxide permeability of 40-80 Barrers; (4) a nitrogen permeability of 10-100 Barrers, and (5) a water vapor transmission rate of not more than 20 (g mil/645cm² (100 in²) day). Optionally, the film should have an optical clarity of between about 0.1% to about 10% as measured by a Hazometer in accordance with ASTM D1003. For adherent cell culture, preferably, the growth surface should have a positive or a negative greater than 4.0 x 10⁻⁴ N/cm (40 dynes/cm). This charge will be referred to as surface energy.

For an explanation of test method ASTM D-790 see "D790-96a Standard Test Methods for Flexural Properties of Unreinforced and Reinforced Plastic and Electrical Insulating Materials" and for an explanation of test method ASTM-D1003 see "D1003-95 Standard Test Method for Haze and Luminous Transmittance of Transparent Plastics". Both explanations are published in the Annual Book of ASTM standards.

The present invention also provides a flexible, gas-permeable cell culture container constructed from the above described films, with the polystyrene layer forming the inner surface of the container.

Another aspect of the present invention provides a flexible, gas permeable cell culture container whose gas permeability may be adjusted to best match the requirements of the cell being cultured in the container. The multi-layer structure of the present film allows one to vary the material of the second layer or substrate layer and its thickness to achieve the desired or predetermined gas permeability requirements for cell growth. Preferably, the type and thickness of the substrate layer and the thickness of the polystyrene layer will be selected to optimize cell growth.

Another aspect of the invention provides for various embodiments of culture containers some of which are advantageous for growing adherent cells, non-adherent cells, and both.

Another aspect of the invention is to provide a flexible, gas permeable cell culture container having a first side that is suitable for growing adherent cells, a second side for growing non-adherent cells, and indicia on the container for indicating the first side from the second side.

### Brief Description of the Drawings

Figure 1 is a cross-sectional view of a two-layer, gas-permeable, flexible film of the invention;
Figure 2 is a cross-sectional view of the flexible, gas-permeable cell culture container of the invention;
Figure 3 is a cross-sectional view of a three-layer, gas-permeable, flexible film of the invention;
Figure 4 is a cross-sectional view of a four-layer, gas-permeable, flexible film of the invention;
Figure 5 is a cross-sectional view of a five-layer, gas-permeable, flexible film of the invention;
Figure 6 is a cross-sectional view of a flexible, gas permeable container for the growth of non-adherent cells;
Figure 7 is a cross-sectional view of a flexible, gas permeable container for the growth of both adherent and non-adherent cells;
Figure 7a is a plan view of the container of Figure 7 showing geometric indicia to distinguish the adherent side from the non-adherent side;
Figure 8 is a cross-sectional view of a flexible, gas permeable container for the growth of non-adherent cells having a clear panel for inspection of the contents;
Figure 9 is a plan view of a flexible, gas permeable container of the present invention having access ports;
Figure 10a is a cross-sectional view of a four layered film of the present invention having a crystalline polystyrene and a high impact polystyrene;
Figure 10b is a cross-sectional view of a three layered film of the present invention having a skin layer;
Figure 11a is a perspective view of a container having a skirt attaching the outer surface of one container panel to the outer surface of an opposite container panel;
Figure 11b is a perspective view of a container formed from two opposed panels each having a polystyrene inner layer deposited on a central portion of substrate layers to leave a peripheral portion of the substrate layers uncoated with polystyrene to form a strong seam.

### Detailed Description of the Invention

The present invention provides a multi-layer, gas-permeable flexible film, having a surface layer formed of polystyrene, and a cell culture container constructed therefrom, having an inner surface of polystyrene.

### I. The Film Components

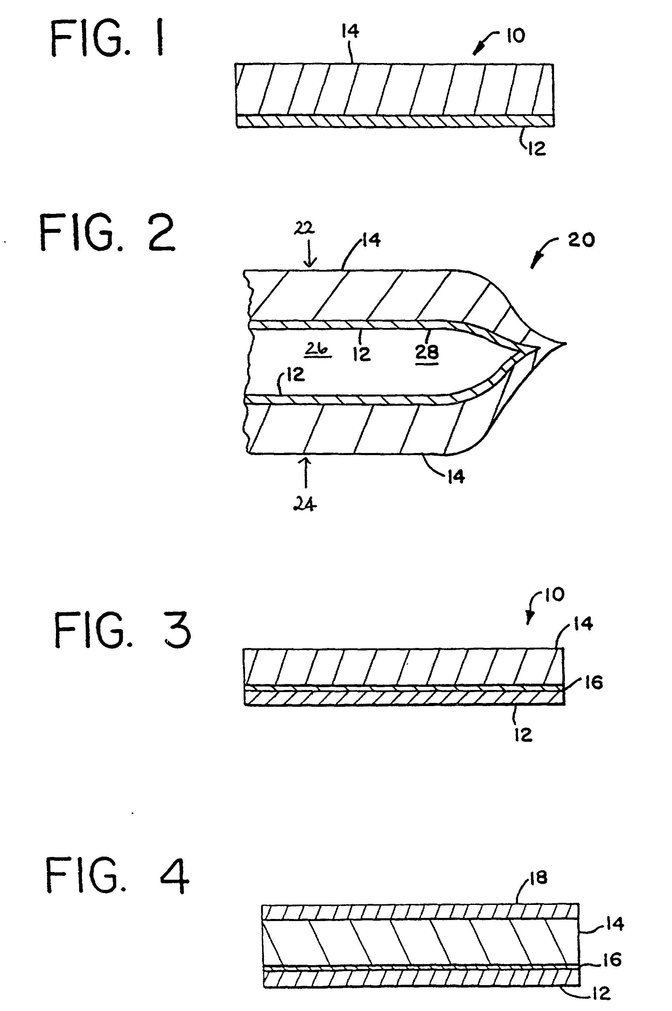

Figure 1 shows a two-layer film 10, of which the first layer 12 of the film forms an inner cell growth surface when fabricated into a cell culture container 20 (Figure 2). The film 10 has an effective thickness to allow cell culture growth. The first layer 12 is an ultra-thin layer of polystyrene, preferably having a thickness from about 2.54µm (0.0001 inches) to about 25.4µm (0.0010 inches), more preferably 5.08µm (0.0002 inches) to about 15.24µm (0.0006 inches), and most preferably about 10.16µm (0.0004 inches). The polystyrene material may be selected from, but not limited to, polystyrenes such as high impact polystyrenes ("HIPS") which are a general purpose polystyrene modified by polybutadiene rubber. Such a polystyrene is sold by Dow Chemical Company under the product designation Styron 47827, Natural. It is also possible to impact modify styrene using styrene-butadienestyrene block copolymers which may be referred to as K-Resin. Crystalline polystyrenes meeting USP Class 6 criteria may also be employed, as may multiple layers of crystalline styrene and impact modified polystyrene.

The second layer 14 is composed of a polymer material such as a polyolefin or a mixture of polymer materials. Preferably, the second layer 14 includes a polymer alloy comprising three components: styrene-ethylene-butadienestyrene ("SEBS") block copolymer (40%-85% by weight), ethylene vinyl acetate (0-40% by weight), and polypropylene (10%-40% by weight) as described in the commonly assigned U.S. Patent No. 4,140,162. Such a polymer alloy is identified by Baxter International Inc. under the product designation PL-732® and fabricated into flexible containers under the tradename Lifecell®. The present invention contemplates using a plurality of layers of the same material or different material to make up the substrate layer. It is also desirable to use, for the second layer 14, other three and four component polymer alloys such as those disclosed in co-pending, and commonly assigned patent application serial number W09514739. One such group of polymer alloys consists of a first component of a polypropylene which constitutes approximately 30-60% by weight of the polymer alloy. The second component is preferably an ultra low density polyethylene or polybutene-1 which constitute approximately 25-50% by weight of the polymer alloy. The third component of the polymer alloy is preferably a dimer fatty acid polyamide (which should be interpreted to include their hydrogenated derivatives as well), which constitutes approximately 5-40% by weight of the polymer alloy. The fourth component is a compatibilizing polymer that may be selected from various block copolymers of styrene with dienes or alpha olefins; the compatibilizing polymers may be modified with minor amounts of chemically active functionalities such as maleic anhydride. For example, the compatibilizing polymer may be an SEBS block copolymer. The fourth component should constitute between 5-40% by weight of the polymer alloy.

Preferably, the second layer 14, (which may sometimes be referred to as the substrate layer 14) has a thickness within a range of about 101.6µm (0.004 inches) to about 635µm (0.025 inches), more preferably 127µm (0.005 inches) to about 304.8µm (0.012 inches), and most preferably 152.4µm (0.006 inches) to about 203.2µm (0.008 inches). It is also possible to use multiple layers of the same substrate, such a PL-732®, material to compose the substrate layer 14. Even if multiple layers are used in the second layer 14, the combined thicknesses of the substrate layer components should still fall within the above-specified thickness ranges.

In another embodiment of the invention (Figure 3), the film may include a first tie layer 16 interposed between the first and second layers 12 and 14. Preferably the first tie layer is a gas permeable olefin and more preferably an ethylene polymer containing vinyl acetate within the range of 16%-30% by weight and most preferably ethylene vinyl acetate with 28% vinyl acetate. Other examples of these polymers include those sold by Quantum Chemicals under the trade name Bynel. It is also possible to use SEBS block copolymers as the first tie layer such as those sold by Shell Chemical Company under the tradename Kraton.

The first tie layer 16 adheres the first layer 12 to the second layer 14. The first tie layer 16 has a thickness preferably within a range from about 5.08µm (0.0002 inches) to about 30.48µm (0.0012 inches0, more preferably from about 10.16µm (0.0004 inches) to about 25.4µm (0.0010 inches), and most preferably about 12.7µm (0.0005 inches).

In yet another embodiment of the invention (Figure 4), the film shown in Figure 3 may also have a skin layer 18 adhered to the second layer 14 opposite the first layer 12, to form an outer skin which adds strength and scratch resistance to the film 10. The skin layer 18 is preferably formed from homo and copolymers of polypropylene, more preferably polypropylene polymers modified by rubber. Such polymers would include those sold by Mitsui under the trade name Admer™. The skin layer 18 preferably has a thickness within a range of from about 2.54µm (0.0001 inches) to about 50.8µm (0.002 inches), and more preferably 12.7µm (0.0005 inches). The skin layer could also be composed of a polyethylene.

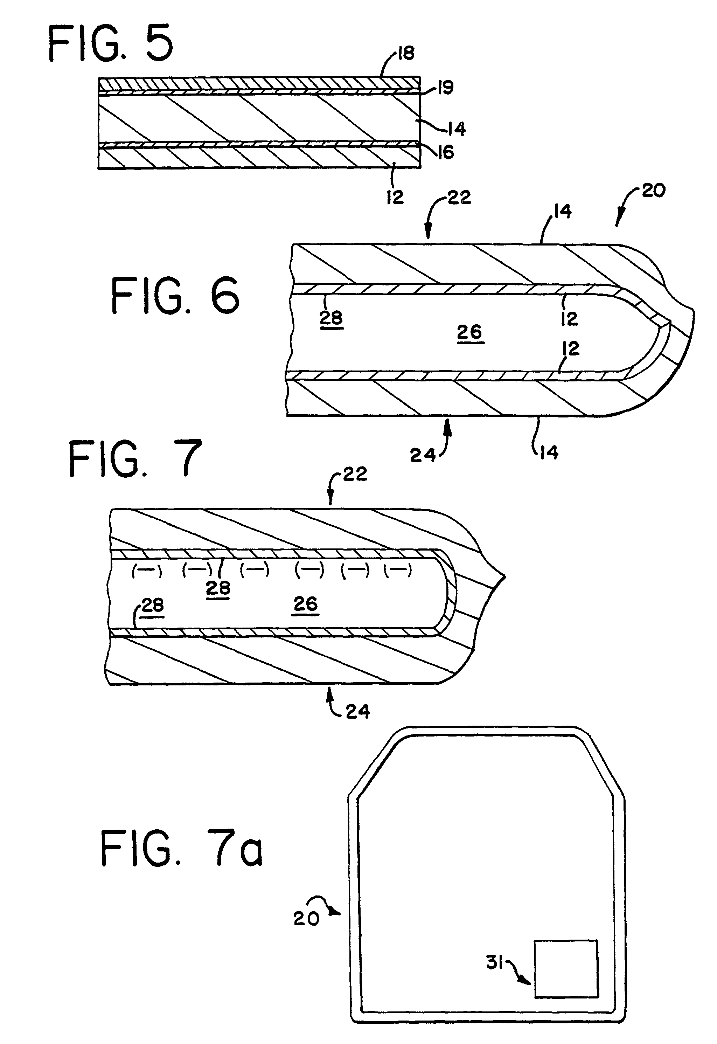

Figure 5 shows the film of figure 4 except with a second tie layer 19 interposed between the skin layer 18 and the substrate layer 14 to adhere the skin layer 18 to the substrate layer 14. The second tie layer 19 may be composed of similar components as identified for the first tie layer such as modified polyethylenes.

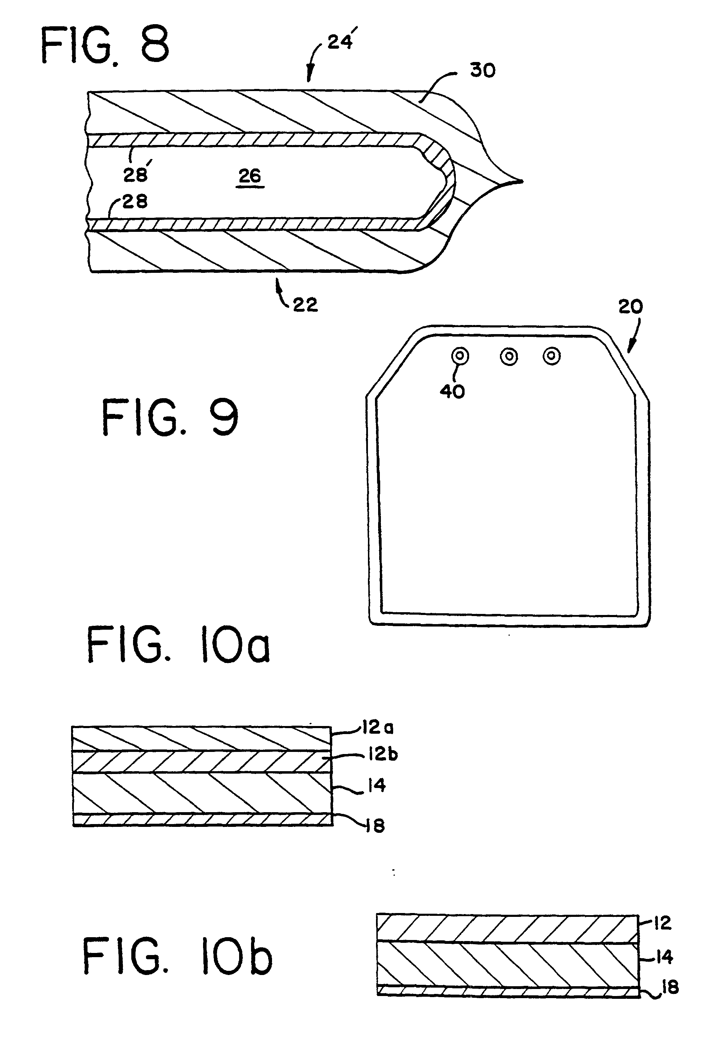

Figure 10a shows yet another film embodiment having four layers. The layers include a layer of crystalline polystyrene 12a, a layer of HIPS 12b, an ethylene vinyl acetate (having a vinyl acetate content between 16%-30%) substrate layer 14, and a polyethylene skin layer 18. The crystalline polystyrene 12a readily accepts and holds a surface energy. The thicknesses of each of the four layers should be consistent with that set forth above with the exception that the combined thickness of the crystalline polystyrene and HIPS should be within the range of thicknesses set forth for the first layer 12. The layers 12a and 12b may be of the same or different thicknesses to form respectively symmetrical or assymmetrical stacks.

Figure 10b shows the film of Figure 10a but without the crystalline polystyrene layer. Thus, the film of Figure 10b has a first layer 12 of HIPS, a substrate layer 14 of EVA and a skin layer 18 of polyethylene. A two layered film such as the one shown in Figure 1 is also contemplated by the present invention having a substrate layer 14 of EVA and a first layer 12 of HIPS.

### II. Construction of the Film and its Physical Characteristics

In forming the film 10 of Figure 1, the ultra-thin layer of polystyrene 12 is co-extruded on the substrate layer 14 using a typical feedblock co-extrusion method.

The resultant film 10 should have a flexural modulus preferably within the range of 3.4 x 10⁷ to 2.1 x 10⁹ Pa (5,000-300,000 psi), more preferably within the range of 6.9 x 10⁷ to 1.4 x 10⁹ Pa (10,000-200,000 psi), and most preferably 6.9 x 10⁷ to 2.1 x 10⁸ Pa (10,000-30,000) psi as measured in accordance with ASTM D 790. The film should have an oxygen permeability within the range of 7-30 Barrers, more preferably 8-20 Barrers, and most preferably 9-15 Barrers. A Barrer has units of (volume of gas in cm³) (film thickness in cm) (1 x 10⁻¹⁰) / (time in seconds) (surface area of film in cm²) (partial pressure of gas in cm of Hg). The film should have a carbon dioxide permeability within the range of 40-80 Barrers. The film should have a nitrogen permeability of 10-100 Barrers. The film 10 should have a water vapor transmission rate of not more than 20 (g mil/100 in²/day). Preferably the film should have an optical clarity within the range of about 0.1%-10% as measured by a Hazometer in accordance with ASTM D1003. For adherent cell culture, preferably, the growth surface should have a surface energy of greater than 4.0 x 10⁻⁴ N/cm (40 dynes/cm). Most adherent cells require a negatively charged surface; however, some adherent cells require a positively charged surface. The containers must also be capable of withstanding radiation sterilization at radiation levels commonly used in the industry for sterilization.

### III. Fabrication of Flexible, Gas Permeable Cell Culture Containers

We turn now to the gas-permeable, flexible cell culture container (20, Figure 2) formed from the multi-layer films described above. The cell culture container 20 includes a body that is constructed from a first side wall 22 and a second side wall 24. The side walls 22 and 24 are sealed along their edges to define a containment area 26 for containing the cell culture media and cells. The side walls 22 and 24 may be sealed by any conventional means such as using heated die and platen which may be followed by a chill die and platen as is well known in the industry. Also, the side walls 22 and 24 may be sealed using inductive welding which also is known in the industry. For containers constructed from films having as the substrate layer 14 the polymer alloy including the dimer fatty acid polyamide, radio frequency techniques may be used. However, the present invention should not be construed to be limited to using any one of these fabrication techniques unless otherwise specified in the claims.

It is possible to construct various flexible, gas permeable containers from the above film in conjunction with other materials.

It has been found that particularly strong seals may be achieved by using the sealing methods disclosed in US-5,935,847.

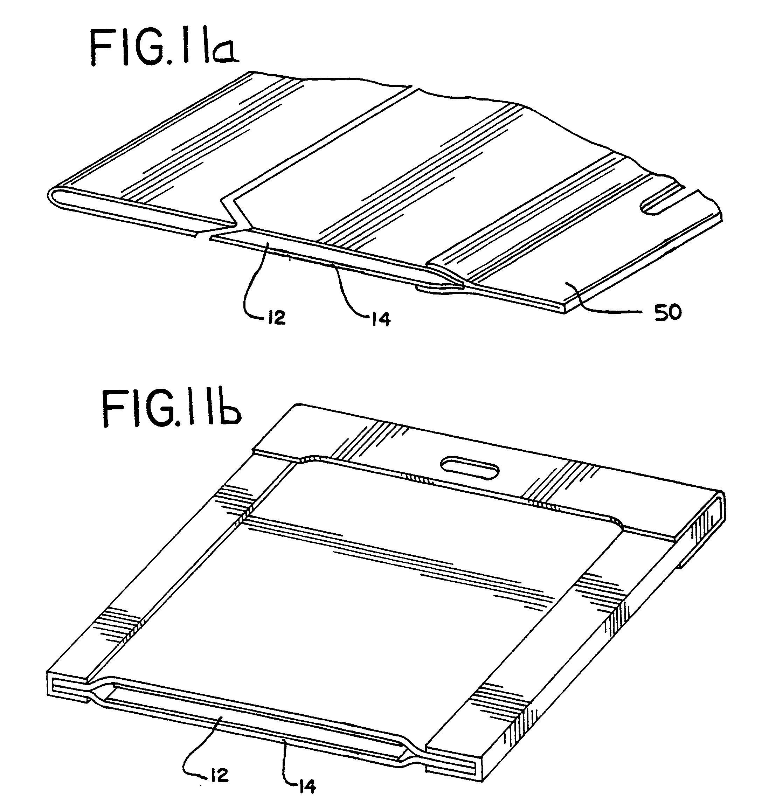

In particular, as shown in Figures 11a and 11b, substrate layers of two films are attached to one another without attaching the polystyrene layers 12 to one another. As is shown in Figure 11a, a skirt 50, composed of the same material as the substrate layer 14, attaches the respective outer surfaces of each of the layers 14 to one another. Figure 11b shows a container constructed from a film having components as described above except the polystyrene layer 12 is deposited on a central portion of the substrate layer 14 leaving a marginal portion of the substrate layer 14 uncoated by the polystyrene. The container is formed by attaching the marginal edges together to form a seam of substrate material bonded to substrate material.

Cell culture containers 20 fabricated using these preferred methods have been found to be sufficiently strong to withstand centrifuging even over an extended period of time at high gravitational forces.

### A. The Non-Adherent Cell Culture Container

Figure 6 shows a flexible, gas permeable cell culture container 10 especially useful for the growth of non-adherent cells. The container 10 is constructed from folding and sealing the substrate layers 14 of the film shown in Figure 1 to define a containment area 26. The first layer of polystyrene 12 faces the containment area 26.

### B. The Hybrid Cell Culture Bag

Figure 7 shows a flexible, gas permeable cell culture container 10 which is suitable for the growth of both adherent and non-adherent cell types. This container is essentially the same as the container set forth in Figure 6 except the first side wall 22 inner surface 28 is charged with a surface energy of greater than 4.0 x 10⁻⁴ N/cm (40 dynes/cm) and preferably about 6.0 x 10⁻⁴ N/cm (60 dynes/cm). The charge shown is negative; however, it could have a positive charge depending upon the type of cell to be cultered. The first side wall 22 with the charge is suitable for growing adherent cells and the second side wall 24 is suitable for growing non-adherent cells. It is desirable to use some indicia 31 to indicate the charged side from the uncharged side such as the perimeter geometry of the cell culture container (See Figure 7a). This would include such structural features as a rounding of corners or notching of any portion of the container, or in any way varying the shape or structural features of the container to indicate the charged side from the uncharged side. It is also possible to have a raised or embossed area on one side of the container. It is also possible to use color coding or other printed indicia for distinguishing the charged and uncharged sides 22 and 24.

### C. Non-Adherent Cell Culture Container with a Clear Panel

Figure 8 shows a flexible, gas permeable cell culture container having a first side wall 22 constructed from the film shown in Figure 1. The second side wall 24' is constructed from a film having a substrate 30 of ethylene vinyl acetate having a vinyl acetate content of 18% ± 2% and an inner layer 28' of HIPS. The first and second side walls 22 and 24' are bonded along their edges as set forth above or by any suitable method. The second side wall 24' has an optical clarity as measured by a Hazometer in accordance with ASTM D1003 within the range of 0.1%-10% which provides for ease of viewing the cells using a microscope or with the naked eye. The first side wall 22 would serve as the cell growth surface for non-adherent cells. It is also possible to apply a surface energy to the inner surface 28 of the side wall 22 to provide for a growing surface for adherent cells.

Preferably each of the containers 20, shown in Figures 6-8, will include access ports 40 as shown in Figure 9. The access ports 40 facilitate filling and emptying of the container 20 of cells or cell culture media without interrupting the closed system. Of course, any number of access ports can be provided as well as a tube set assembly, or the like.

### D. Other Containers

It is also desirable to construct containers using the films shown in Figures 3-5 and 10a-10b.

### IV. Method of Providing Adjustable Gas Permeability

It is desirable to construct a flexible cell culture container 20 having a predetermined gas permeability. The predetermined gas permeability selected promotes cell growth within the container 20. Preferably, the selected permeability optimizes cell growth.

The gas permeability depends upon the types of polyolefin second layer, the thickness of the individual layers, and the overall thickness of the film.

Thus, the method of constructing or fabricating a gas permeable, cell growth container having a predetermined gas permeability comprises the following steps: providing a polystyrene, providing an appropriate polyolefin substrate layer, and coextruding the polystyrene and the polymer producing a layered film having a gas permeability to effect cell growth. Preferably, the cell growth will be optimal.

### Example 1

Films in accordance with the present invention were coextruded from a 7.62µm (0.0003 inch) thick layer of polystyrene (K-Resin or HIPS) on a 190.5µm (0.0075 inch) thick layer of polyolefin alloy (PL-732® or PL-269® ). A portion of the 732® /HIPS film was corona discharge treated (good results have also been achieved without corona discharge as shown in Table 1 below). The film was formed into a flexible container or bag using a heat seal process. A length of film was cut from a roll of coextruded film. Port fitments, described in the commonly assigned U.S. Patent

No. 4,327,726, which is incorporated herein by reference, were heat sealed to the film near the midpoint of the length. The film was folded across the width of the sheet, near the sealed port fitments. The folded sheet with port fitments was placed on a heated brass platen and heat sealed using a heated brass die. The die and platen were operated at a constant temperature, the die at 138°C (280°F), and the platen at 188°C (370°F). No chilling dies or devices were employed. After sealing the container, the container was removed from the platen and allowed to air cool. Port closures were solvent bonded to the port fitments as is known in the art.

The bags were radiation sterilized and employed in cell culture studies of human progenitor cells. The bags were used to culture progenitor cells in a 10-12 day period in vitro culture. Progenitor cells were derived from purified CD34+ cells (stem cells) collected from peripheral blood as mobilized stem cells during a leukopheresis procedure. The cells were cultured via a process described in US-5,700,691 . Coextruded cell culture bags were seeded at a cell density of 0.1-1x10⁵ (cells/ml). Coextruded culture bags were able to support an increase in the total number of viable cells during the 10-12 day culture period. Typical increases of 40-70 fold were observed. Cell proliferation in excess of 100 fold has been obtained in the culture bags using recombinant growth factors known to support progenitor cell growth in vitro. In addition to the increase in cell number, the culture bags were able to support an increase in Colony Forming Cells ("CFC") and early granulocytes as determined by flow cytometry and cytological staining. Early granulocytes are considered nonadherent cells that grow well in an adherent cell environment.

### Example 2

A film in accordance with the present invention was coextruded with a 7.62µm-12.7µm (0.0003-0.0005 inch) thick layer of high impact modified polystyrene on a 190.5µm-203.2µm (0.0075- 0.0080 inch) thick layer of polyolefin alloy (PL-732® ). The film was formed into a flexible container or bag using a heat seal process. The flexible container was inserted into an envelope composed of PL-732® of between 0.025cm-0.028cm (0.010-0.011 inches) thick, and the envelope was heat sealed to the container around the periphery.

The composite container was used to culture progenitor cells over a three day period in vitro culture. Progenitor cells were derived from purified CD34+ cells (stem cells) collected from peripheral blood as mobilized stem cells during a leukopheresis procedure. The cells were cultured via a process described in co-pending and commonly assigned patent application Serial No. . Coextruded composite containers were seeded at a cell density of 1x10⁵ (cells/ml). After three days the culture density was 19.5x10⁵ (cell/ml) with 82% of the CD 34+ cells viable.

These growth data compare favorably to a container made in accordance with Example 1 which, in the same experiment, had a culture density of 17.8x 10⁵ (cell/ml) at the end of the three day culture period.

### Example 3

Films made in accordance with the present invention were coextruded and were used to culture progenitor cells over a ten day period in vitro culture (X-Vivo 10 + 10 mg/ml HSA with IL3, G-CSF, GM-CSF at 1000 U/ml each and SCF at 20 ng/ml). Progenitor cells were derived from purified CD34+ cells (stem cells) collected from peripheral blood as mobilized stem cells during a leukopheresis procedure. The cell proliferation data for various containers are set forth in the Tables 1 and 2 for two separate experiments.

**TABLE 1**

| Bag Type and Treatment | Total Cells | P.I.* | % Viable | Viable Cells |
|---|---|---|---|---|
| PL-269**+K-Resin | 6.3E+06 | 21 | 75.85 | 4.8E+06 |
| PL-732+K-Resin | 2.1E+07 | 71 | 85.87 | 1.8E+07 |
| PL-732+HIPS | 3.3E+07 | 111 | 87.08 | 2.9E+07 |
| PL-732+HIPS/CD*** | 3.1E+07 | 102 | 82.89 | 2.5E+07 |
| TEFLON® | 3.4E+07 | 114 | 86.16 | 2.9E+07 |
| Polystyrene Control | 4.0E+07 | 132 | 82.24 | 3.3E+07 |

**TABLE 2**

| Bag Type and Treatment | Total Cells | P.I.* | % Viable | Viable Cells |
|---|---|---|---|---|
| PL-269**+K-Resin | 1.2E+07 | 20 | 94.26 | 1.1E+07 |
| PL-732+K-Resin | 1.5E+07 | 26 | 95.39 | 1.5E+07 |
| PL-732+HIPS | 2.1E+07 | 35 | 96.01 | 2.0E+07 |
| PL-732+HIPS/CD*** | 2.4E+07 | 40 | 95.88 | 2.3E+07 |
| TEFLON® | 2.3E+07 | 39 | 94.68 | 2.2E+07 |
| Polystyrene Control | 2.7E+07 | 46 | 94.39 | 2.6E+07 |

| | | | | |
|---|---|---|---|---|
| * P.I. stands for Proliferation Index which indicates the fold increase in the cell growth. Thus, a proliferation index of 2 indicates a doubling in the number of cells. | | | | |
| **PL 269 is EVA (vinyl acetate 18%). | | | | |
| *** CD stands for corona discharge treated. | | | | |

### Example 4

Containers were coextruded from PL-732® and HIPS as set forth in Example 1. PL-732® containers and Teflon® containers are commercially available. These containers were used to culture lymphokine activated killer cells over a seven day period in AIM-V (serum free culture media) with IL-2 1000 U/ml growth factor. The results of this experiment are set forth in Table 3 wherein P.I. ± S.D. stands for proliferation index plus or minus an indicated standard deviation.

**TABLE 3**

| | |
|---|---|
| Bag Type and Treatment | P.I. ± S.D. |
| PL-732® + HIPS | 3.0 ± 1.7 |
| PL-732® | 1.6 ± 1.2 |
| TEFLON® | 3.6 ± 2.6 |

### Example 5

Containers were coextruded from PL-732® and HIPS as set forth in Example 1. PL-732® containers and Teflon® containers are commercially available. These containers were used to culture tumor infiltrating lymphocytes over a seven day period in AIM-V (serum free culture media) with IL-2 1000 U/ml growth factor. The results of this experiment are set forth in Table 4 wherein P.I. ± S.D. stands for proliferation index plus or minus an indicated standard deviation.

**TABLE 4**

| | |
|---|---|
| Bag Treatment | P.I. ± S.D. |
| PL-732® + HIPS | 9.0 ± 2.7 |
| PL-732® | 9.5 ± 5.7 |
| TEFLON® | 2.8 ± 2.0 |

## Claims

1. A multi-layer, flexible, gas-permeable film suitable for forming a cell culture container, the film comprising:
a first layer composed of a polystyrene having a thickness within the range of 2.54 µm (0.0001) inches to about 25.4 µm (0.0010 inches); and,
a second layer adhered to the first layer composed of a polymer alloy having a thickness within the range of 101.6 µm (0.004 inches) to about 635 µm (0.025 inches).

2. The film of claim 1 wherein the polystyrene of the first layer is modified by polybutadiene rubber.

3. The film of claim 2 further including a third layer of crystalline polystyrene attached to the first layer on a side opposite the second layer.

4. The film of claim 1 wherein the first layer has a thickness in the range of about 5.08 µm (0.0002 inches) to about 20.32 µm (0.0008 inches).

5. The film of claim 1 wherein the first layer has a thickness of about 10.16 µm (0.0004 inches).

6. The film of any preceding claim further comprising a layer of polyethylene on the second layer on a side opposite the first layer.

7. The film of any one of claims 1 to 5 further comprising a skin layer attached to the second layer on a side opposite the first layer.

8. The film of claim 7 wherein the skin layer has a thickness within the range of 2.54 µm (0.0001 inches) to about 50.8 µm (0.002 inches).

9. The film of claim 8 wherein the skin layer has a thickness of about 12.7 µm (0.0005 inches).

10. The film of claim 7, 8 or 9 wherein the skin layer is selected from polypropylene and polyethylene.

11. The film of any preceding claim further comprising a tie layer interposed between the first and second layers, the tie layer providing adhesive compatibility between the first and second layers.

12. The film of claim 11 wherein the tie layer is composed of a gas permeable olefin.

13. The film of claim 12 wherein the gas permeable olefin is an ethylene polymer containing vinyl acetate within the range of 16%-32% by weight.

14. The film of claim 13 wherein the ethylene vinyl acetate has a vinyl acetate content of 28% by weight.

15. A multi-layer, flexible, gas-permeable film suitable for forming a cell culture container, the film comprising:
a first layer composed of a polystyrene having a thickness within the range of 2.54 µm (0.0001 inches) to about 25.4 µm (0.0010 inches);
a second layer adhered to the first layer composed of a polymer alloy; and,
wherein the film having physical properties within the range:
a > 6.9 x 10⁷ but < 2.1 x 10⁸ Pa > 10,000 but < 30,000 psi)
b > 9 but < 15
c > 40 but < 80
d > 10 but < 100
e < 20
wherein:
a is the flexural modulus of the film measured according to ASTM D-790;
b is the oxygen permeability in Barrers;
c is the carbon dioxide permeability in Barrers;
d is the nitrogen permeability in Barrers; and
e is the water vapor transmission rate in g mil/ 645cm² (100 in²) /day).

16. The film of claim 15 wherein the film has an optical clarity within the range of 0.1%-10% as measured by a Hazometer under ASTM D1003.

17. The film of clam 15 or 16 wherein the second layer has a thickness within the range of 101.6 µm (0.004 inches) to about 635 µm (0.025 inches).

18. The film of any preceding claim wherein the polymer alloy has three components, a first component is a styrene-ethylene-butadiene-styrene block copolymer, a second component is ethylene vinyl acetate, and a third component is polypropylene.

19. The film of claim 18 wherein the styreneethylene-butadiene-styrene block copolymer constitutes 40%-85% by weight of the polymer alloy, the ethylene vinyl acetate constitutes 0 to 40% by weight of the polymer alloy, and the polypropylene constitutes 10 to 40% by weight of the polymer.

20. The film of any one of claims 1 to 17 wherein the polymer alloy has four components.

21. The film of claim 20 wherein the four component polymer alloy has a first component of a polypropylene, a second component selected from the group consisting essentially of an ultra low density polyethylene and polybutene-1, a third component of a dimer fatty acid polyamide, and a fourth component of a styrene-ethylenebutadiene-styrene block copolymer.

22. The film of claim 21 wherein the first component constitutes within the range of 30-60% by weight of the polymer alloy, the second component constitutes within the range of 25%-50% by weight of the polymer alloy, the third component constitutes within the range of 5%-40% by weight of the polymer alloy, and the fourth component constitutes 5%-40% by weight of the polymer alloy.

23. The film of any one of claims 1 to 17 wherein the first layer has a surface energy greater than 4.0 x 10⁻⁴ N/cm (40 dynes/cm).

24. A flexible, gas-permeable cell culture container suitable for culturing cells, the container comprising:
a first and second side wall each having edges, the first and second side walls being sealed together at their respective side wall edges to provide a containment area, wherein at least the first side wall is composed of a film according to claim 1 having a first layer of polystyrene having a thickness within the range of 2.54 µm (0.0001 inches) to about 25.4 µm (0.0010 inches), and, a second layer adhered to the first layer of a polymer alloy having a thickness within the range of 10.16 µm (0.004 inches) to about 635 µm (0.025 inches).

25. The cell culture container of claim 24 further comprising at least one access port for accessing the containment area.

26. The cell culture container of claim 24 or 25 wherein the first layer has a surface energy greater than 4.0 x 10⁻⁴ N/cm (40 dynes/cm).

27. A method for fabricating a multi-layered film according to claim 1 for culturing cells comprising the steps of:
providing a polystyrene;
providing a polymer alloy; and,
coextruding the polystyrene and the polymer material producing a layered film having a predetermined gas permeability to promote cell growth.

28. The method of claim 27 wherein the polystyrene layer has a thickness within the range of about 2.54 µm (0.0001 inches) to about 25.4 µm (0.0010 inches), and wherein the polymer alloy has a thickness within the range of about 10.16 µm (0.004 inches) to about 635 µm (0.025 inches).

29. A flexible, gas-permeable cell culture container according to claim 24 suitable for culturing cells, the container comprising:
a first side wall of the container being suitable for growing adherent cells;
a second side wall attached to the first side wall for growing non-adherent cells; and,
means associated with the container for distinguishing the first side wall from the second side wall.

30. The container of claim 29 wherein the means for distinguishing the first side wall from the second side wall is the geometry of the container.

31. The container of claim 29 or 30 wherein the first sidewall of the container has a surface energy greater than 4.0 x 10⁻⁴ N/cm (40 dynes/cm).

## Patentansprüche

1. Flexible, gasdurchlässige Mehrschichtfolie, die zum Formen eines Zellkulturbehälters geeignet ist, wobei die Folie folgendes aufweist:
eine erste Schicht, die aus einem Polystyrol besteht und eine Dicke im Bereich von 2,54 µm (0,0001 inch) bis etwa 25,4 µm (0,0010 inch) hat; und
eine zweite Schicht, die an der ersten Schicht haftet und aus einer Polymerlegierung besteht, die eine Dicke im Bereich von 101,6 µm (0,004 inch) bis etwa 635 µm (0,025 inch) hat.

2. Folie nach Anspruch 1,
wobei das Polystyrol der ersten Schicht mit einem Polybutadienkautschuk modifiziert ist.

3. Folie nach Anspruch 2,
die ferner eine dritte Schicht aus einem kristallinen Polystyrol aufweist, die auf der ersten Schicht auf einer Seite angebracht ist, die der zweiten Schicht gegenüberliegt.

4. Folie nach Anspruch 1,
wobei die erste Schicht eine Dicke im Bereich von etwa 5,08 µm (0,0002 inch) bis etwa 20,32 µm (0,0008 inch) hat.

5. Folie nach Anspruch 1,
wobei die erste Schicht eine Dicke von etwa 10,16 µm (0,0004 inch) hat.

6. Folie nach einem der vorhergehenden Ansprüche,
die eine Schicht aus Polyethylen auf der zweiten Schicht auf einer Seite aufweist, die der ersten Schicht gegenüberliegt.

7. Folie nach einem der Ansprüche 1 bis 5,
die ferner eine Hautschicht aufweist, die auf der zweiten Schicht auf einer Seite angebracht ist, die der ersten Schicht gegenüberliegt.

8. Folie nach Anspruch 7,
wobei die Hautschicht eine Dicke im Bereich von 2,54 µm (0,0001 inch) bis etwa 50,8 µm (0,002 inch) hat.

9. Folie nach Anspruch 8,
wobei die Hautschicht eine Dicke von etwa 12,7 µm (0,0005 inch) hat.

10. Folie nach Anspruch 7, 8 oder 9,
wobei die Hautschicht aus Polypropylen und Polyethylen ausgewählt ist.

11. Folie nach einem der vorhergehenden Ansprüche,
die ferner eine Verbindungsschicht aufweist, die zwischen der ersten und der zweiten Schicht angeordnet ist, wobei die Verbindungsschicht für Haftkompatibilität zwischen der ersten und zweiten Schicht sorgt.

12. Folie nach Anspruch 11,
wobei die Verbindungsschicht aus einem gasdurchlässigen Olefin besteht.

13. Folie nach Anspruch 12,
wobei das gasdurchlässige Olefin ein Ethylenpolymer ist, das Vinylacetat im Bereich von 16 bis 32 Gew.-% enthält.

14. Folie nach Anspruch 13,
wobei das Ethylenvinylacetat einen Vinylacetatgehalt von 28 Gew.-% hat.

15. Flexible, gasdurchlässige Mehrschichtfolie, die zum Formen eines Zellkulturbehälters geeignet ist, wobei die Folie folgendes aufweist:
eine erste Schicht, die aus einem Polystyrol besteht und eine Dicke im Bereich von 2,54 µm (0,0001 inch) bis etwa 25,4 µm (0,0010 inch) hat;
eine zweite Schicht, die an der ersten Schicht haftet und aus einer Polymerlegierung besteht; und
wobei die Folie physikalische Eigenschaften in folgenden Bereichen hat:
a > 6,9 x 10⁷, aber < 2,1 x 10⁸ Pa (> 10.000, aber < 30.000 psi)
b > 9, aber < 15
c > 40, aber < 80
d > 10, aber < 100
e < 20
wobei
a = Biegemodul der Folie, gemessen nach ASTM D-790;
b = Sauerstoffdurchlässigkeit in Barrer;
c = Kohlendioxiddurchlässigkeit in Barrer;
d = Stickstoffdurchlässigkeit in Barrer; und
e = Wasserdampfdurchlaßgrad in g mil/645 cm²/Tag.

16. Folie nach Anspruch 15,
wobei die Folie eine optische Klarheit im Bereich von 0,1 % bis 10 %, gemessen mit einem Hazometer nach ASTM D1003, hat.

17. Folie nach Anspruch 15 oder 16,
wobei die zweite Schicht eine Dicke im Bereich von 101,6 µm (0,004 inch) bis etwa 635 µm (0,025 inch) hat.

18. Folie nach einem der vorhergehenden Ansprüche,
wobei die Polymerlegierung drei Komponenten hat und wobei eine erste Komponente ein Styrol-Ethylen-Butadien-Styrol-Blockcopolymer ist, eine zweite Komponente Ethylenvinylacetat ist und eine dritte Komponente Polypropylen ist.

19. Folie nach Anspruch 18,
wobei das Styrol-Ethylen-Butadien-Styrol-Blockcopolymer 40 bis 85 Gew.-% der Polymerlegierung ausmacht, das Ethylenvinylacetat 0 bis 40 Gew.-% der Polymerlegierung ausmacht, und das Polypropylen 10 bis 40 Gew.-% des Polymers ausmacht.

20. Folie nach einem der Ansprüche 1 bis 17,
wobei die Polymerlegierung vier Komponenten hat.

21. Folie nach Anspruch 20,
wobei die Vierkomponenten-Polymerlegierung folgendes aufweist: eine erste Komponente aus einem Polypropylen, eine zweite Komponente, die aus der Gruppe ausgewählt ist, die im wesentlichen aus Polyethylen ultraniedriger Dichte und Polybuten-1 besteht, eine dritte Komponente aus einem Dimerfettsäurepolyamid und eine vierte Komponente aus einem Styrol-Ethylen-Butadien-Styrol-Blockcopolymer.

22. Folie nach Anspruch 21,
wobei die erste Komponente einen Anteil von 30 bis 60 Gew.-% der Polymerlegierung ausmacht, die zweite Komponente einen Anteil von 25 bis 50 Gew.-% der Polymerlegierung ausmacht, die dritte Komponente einen Anteil von 5 bis 40 Gew.-% der Polymerlegierung ausmacht, und die vierte Komponente 5 bis 40 Gew.-% der Polymerlegierung ausmacht.

23. Folie nach einem der Ansprüche 1 bis 17,
wobei die erste Schicht eine Oberflächenenergie von mehr als 4,0 x 10⁻⁴ N/cm (40 Dyn/cm) hat.

24. Flexibler, gasdurchlässiger Zellkulturbehälter, der zum Kultivieren von Zellen geeignet ist, wobei der Behälter folgendes aufweist:
eine erste und eine zweite Seitenwand, von denen jede Ränder hat, wobei die erste und die zweite Seitenwand an ihren jeweiligen Seitenwandrändern miteinander verschweißt sind, um einen Umschließungsbereich zu bilden,
wobei zumindest die erste Seitenwand aus einer Folie nach Anspruch 1 besteht, die folgendes aufweist: eine erste Schicht aus Polystyrol, die eine Dicke im Bereich von 2,54 µm (0,0001 inch) bis etwa 25,4 µm (0,0010 inch) hat, und eine an der ersten Schicht haftende zweite Schicht aus einem Polymer, die eine Dicke im Bereich von 10,16 µm (0,004 inch) bis etwa 635 µm (0,025 inch) hat.

25. Zellkulturbehälter nach Anspruch 24,
der ferner mindestens eine Zugangsöffnung für den Zugang zu dem Umschließungsbereich aufweist.

26. Zellkulturbehälter nach Anspruch 24 oder 25,
wobei die erste Schicht eine Oberflächenenergie von mehr als 4,0 x 10⁻⁴ N/cm (40 Dyn/cm) hat.

27. Verfahren zur Herstellung einer Mehrschichtfolie nach Anspruch 1 zum Kultivieren von Zellen, wobei das Verfahren die folgenden Schritte aufweist:
Bereitstellen eines Polystyrols;
Bereitstellen einer Polymerlegierung; und
Coextrudieren des Polystyrol- und des Polymermaterials zur Erzeugung einer Schichtfolie, die eine vorbestimmte Gasdurchlässigkeit hat, um ein Zellwachstum zu fördern.

28. Verfahren nach Anspruch 27,
wobei die Polystyrolschicht eine Dicke im Bereich von etwa 2,54 um (0,0001 inch) bis etwa 25,4 µm (0,0010 inch) hat, und wobei die Polymerlegierung eine Dicke im Bereich von etwa 10,16 µm (0,004 inch) bis etwa 635 um (0,025 inch) hat.

29. Flexibler, gasdurchlässiger Zellkulturbehälter nach Anspruch 24, der zum Kultivieren von Zellen geeignet ist, wobei der Behälter folgendes aufweist:
eine erste Seitenwand des Behälters, die zum Kultivieren anhaftender Zellen geeignet ist;
eine an der ersten Seitenwand angebrachte zweite Seitenwand zum Kultivieren nichtanhaftender Zellen; und
dem Behälter zugeordnete Mittel zum Unterscheiden der ersten Seitenwand von der zweiten Seitenwand.

30. Behälter nach Anspruch 29,
wobei das Mittel zum Unterscheiden der ersten Seitenwand von der zweiten Seitenwand die Geometrie des Behälters ist.

31. Behälter nach Anspruch 29 oder 30,
wobei die erste Seitenwand des Behälters eine Oberflächenenergie von mehr als 4,0 x 10⁻⁴ N/cm (40 Dyn/cm) hat.

## Revendications

1. Film perméable aux gaz, flexible et multicouche, convenant à la formation d'un récipient de culture cellulaire, le film comprenant :
une première couche composée d'un polystyrène ayant une épaisseur située dans la plage allant de 2,54 µm (0,0001 pouce) à environ 25,4 µm (0,0010 pouce) ; et
une deuxième couche collée à la première couche, composée d'un alliage polymère ayant une épaisseur située dans la plage allant de 101,6 µm (0,004 pouce) à environ 635 µm (0,025 pouce).

2. Film selon la revendication 1, dans lequel le polystyrène de la première couche est modifié par un caoutchouc de polybutadiène.

3. Film selon la revendication 2, contenant en outre une troisième couche de polystyrène cristallin rattachée à la première couche sur le côté à l'opposé de la deuxième couche.

4. Film selon la revendication 1, dans lequel la première couche a une épaisseur située dans la plage allant d'environ 5,08 µm (0,0002 pouce) à environ 20,32 µm (0,0008 pouce).

5. Film selon la revendication 1, dans lequel la première couche a une épaisseur d'environ 10,16 µm (0,0004 pouce).

6. Film selon l'une quelconque des revendications précédentes, comprenant en outre une couche de polyéthylène sur la deuxième couche sur le côté à l'opposé de la première couche.

7. Film selon l'une quelconque des revendications 1 à 5, comprenant en outre une couche de peau rattachée à la deuxième couche sur le côté à l'opposé de la première couche.

8. Film selon la revendication 7, dans lequel la couche de peau a une épaisseur située dans la plage allant de 2,54 µm (0,0001 pouce) à environ 50,8 µm (0,002 pouce).

9. Film selon la revendication 8, dans lequel la couche de peau a une épaisseur d'environ 12,7 µm (0,0005 pouce).

10. Film selon la revendication 7, 8 ou 9, dans lequel la couche de peau est choisie parmi le propylène et le polyéthylène.

11. Film selon l'une quelconque des revendications précédentes, comprenant en outre une couche d'attache interposée entre les première et deuxième couches, la couche d'attache conférant une compatibilité adhésive entre les première et deuxième couches.

12. Film selon la revendication 11? Dans lequel la couche d'attache est composée d'une oléfine perméable aux gaz.

13. Film selon la revendication 12, dans lequel l'oléfine perméable aux gaz est un polymère d'éthylène contenant de l'acétate de vinyle à raison de 16 à 32 % en poids.

14. Film selon la revendication 13, dans lequel l'éthylène/acétate de vinyle a une teneur en acétate de vinyle de 28 % en poids.

15. Film perméable aux gaz, flexible et multicouche, convenant à la formation d'un récipient de culture cellulaire, le film comprenant :
une première couche composée d'un polystyrène ayant une épaisseur située dans la plage allant de 2,54 µm (0,0001 pouce) à environ 25,4 µm (0,0010 pouce) ; et
une deuxième couche collée à la première couche, composée d'un alliage polymère ; et
où le film a des propriétés physiques situées dans les plages suivantes :
a > 6,9 x 10⁶ mais < 2,1 x 10⁸ Pa (> 10 000 mais < 30 000 psi)
b > 9 mais < 15
c > 40 mais < 80
d > 10 mais < 100
e < 20
où :
a est le module de flexion du film, mesuré conformément à la norme ASTM D-790 ;
b est la perméabilité à l'oxygène en Barrers ;
c est la perméabilité au dioxyde de carbone en Barrers ;
d est la perméabilité à l'azote en Barrers ; et
e est la vitesse de transmission de vapeur d'eau en g mil/645 cm² (100 in²)/jour.

16. Film selon la revendication 15? Dans lequel le film a une clarté optique située dans la plage allant de 0,1 à 10 %, telle que mesurée par un dispositif de mesure de voile Hazometer selon la norme ASTM D1003.

17. Film selon la revendication 15 ou 16, dans lequel la deuxième couche a une épaisseur située dans la plage allant de 101,6 µm (0,004 pouce) à environ 635 µm (0,025 pouce).

18. Film selon l'une quelconque des revendications précédentes, dans lequel l'alliage polymère a trois composants, un premier composant est un copolymère séquencé de styrène/éthylène/butadiène/styrène, un deuxième composant est un éthylêne/acétate de vinyle, et un troisième composant est le polypropylène.

19. Film selon la revendication 18, dans lequel le copolymère séquencé de styrène/éthylène/butadiène/ styrène représente de 40 à 85 % en poids de l'alliage polymère, l'éthylène/acétate de vinyle constitue de 0 à 40 % en poids de l'alliage polymère, et le polypropylène constitue de 10 à 40 % en poids du polymère.

20. Film selon l'une quelconque des revendications 1 à 17, dans lequel l'alliage polymère a quatre composants.

21. Film selon la revendication 20, dans lequel l'alliage polymère à quatre composants a un premier composant en un polypropylène, un deuxième composant choisi dans l'ensemble constitué principalement d'un polyéthylène ultra basse densité et de polybutène-1, un troisième composant en un polyamide d'acide gras dimère, et un quatrième composant en un copolymère séquencé styrène/éthylène/butadiène/styrène.

22. Film selon la revendication 21, dans lequel le premier composant représente de 30 à 60 % en poids de l'alliage polymère, le deuxième composant représente de 25 à 50 % en poids de l'alliage polymère, le troisième composant constitue de 5 à 40 % en poids de l'alliage polymère et le quatrième composant constitue de 5 à 40 % en poids de l'alliage polymère.

23. Film selon l'une quelconque des revendications 1 à 17, dans lequel la première couche a une énergie de surface supérieure à 4,0 x 10⁻⁴ N/cm (40 dynes/cm).

24. Récipient de culture cellulaire perméable aux gaz, flexible, convenant à la culture de cellules, le récipient comprenant :
une première et une deuxième parois latérales ayant chacune des bords, les première et deuxième parois latérales étant scellées ensemble au niveau de leurs bords de paroi latérale respectifs pour réaliser une zone de confinement, où au moins la première paroi latérale est composée d'un film selon la revendication 1 ayant une première couche de polystyrène ayant une épaisseur située dans la plage allant de 2,54 µm (0,0001 pouce) à environ 25,4 µm (0,0010 pouce), et une deuxième couche collée à la première couche, en un alliage polymère ayant une épaisseur située dans la plage allant de 10,16 µm (0,004 pouce) à environ 635 µm (0,025 pouce).

25. Récipient de culture cellulaire selon la revendication 24, comprenant en outre au moins un orifice d'accès pour accéder à la zone de confinement.

26. Récipient de culture cellulaire selon la revendication 24 ou 25, dans lequel la première couche a une énergie de surface supérieure à 4,0 x 10⁻⁴ N/cm (40 dynes/cm).

27. Procédé pour fabriquer un film multicouche selon la revendication 1 pour cultiver des cellules, comprenant les étapes consistant à :
disposer d'un polystyrène ;
disposer d'un alliage polymère ; et
coextruder le polystyrène et le matériau polymère, produisant un film stratifié ayant une perméabilité aux gaz prédéterminée pour favoriser la croissance des cellules.

28. Procédé selon la revendication 27, dans lequel la couche de polystyrène a une épaisseur située dans la plage allant d'environ 2,54 µm (0,0001 pouce) à environ 25,4 µm (0,0010 pouce), et dans lequel l'alliage polymère a une épaisseur située dans la plage allant d'environ 10,16 µm (0,004 pouce) à environ 635 µm (0,025 pouce).

29. Récipient de culture cellulaire perméable aux gaz, flexible, selon la revendication 24, convenant à la culture de cellules, le récipient comprenant :
une première paroi latérale qu récipient qui convient pour la croissance de cellules adhérentes ;
une deuxième paroi latérale rattachée à la première paroi latérale pour la croissance de cellules non adhérences ; et
des moyens associés au récipient pour faire une distinction entre la première paroi latérale et la deuxième paroi latérale.

30. Récipient selon la revendication 29, dans lequel les moyens pour faire une distinction entre la première paroi latérale et la deuxième paroi latérale sont la géométrie du récipient.

31. Récipient selon la revendication 29 ou 30, dans lequel la première paroi latérale du récipient a une énergie de surface supérieure à 4,0 x 10⁻⁴ N/cm (40 dynes/cm).
